# EUROPEAN PATENT APPLICATION

(11) **EP 2 682 399 A1**
(43) Date of publication of application: **08.01.2014**
(21) Application number: 12752556.6
(22) Date of filing: 28.02.2012
(51) Int. Cl.: C07H 17/08, A61K 31/7048, A61P 31/04

(54) **NOVEL MACROLIDE INTERMEDIATE AND NOVEL PRODUCTION PROCESS**

(30) Priority: 28.02.2011 JP 2011043150
(71) Applicant: Meiji Seika Pharma Co., Ltd., Tokyo 104-8002 (JP)
(72) Inventor: KURIHARA, Kenichi, Yokohama-shi Kanagawa 222-8567 (JP); MITOMI, Masaaki, Yokohama-shi Kanagawa 222-8567 (JP); MINOWA, Nobuto, Yokohama-shi Kanagawa 222-8567 (JP)
(74) Representative: KATZAROV S.A.
(86) International application number: PCT/JP2012/054855
(87) International publication number: WO 2012/118048

(57) **Abstract**

Provided are: a novel monoalkylamino intermediate (I); and a production method for a compound represented by the formula (III), which is an animal antibacterial agent, via the novel monoalkylamino intermediate.

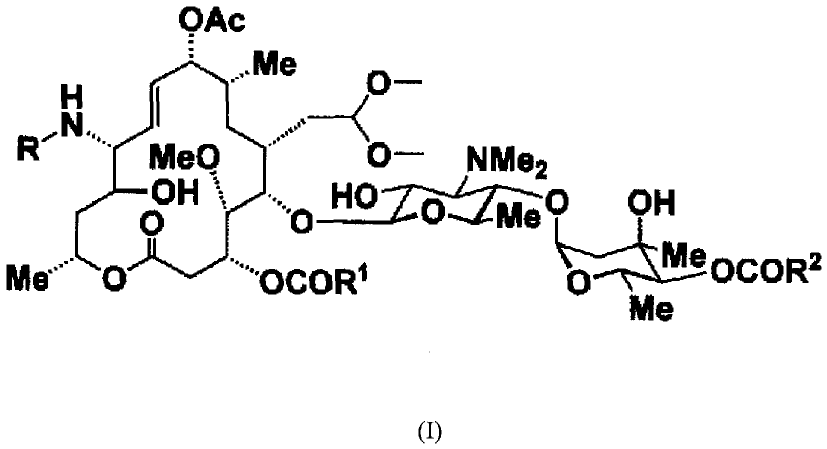

## Description

### TECHNICAL FIELD

The present invention relates to a novel production method for a macrolide derivative effective as a therapeutic drug for bacterial infections in animals.

### BACKGROUND ART

The inventors of the present invention have discovered that derivatives of midecamycin modified at the C-12 and C-13 positions have excellent antibacterial activities (WO 2002/064607 A1). In recent years, the inventors have found that a compound represented by the formula (IIIa) out of the derivatives exhibits remarkably strong antibacterial actions against pathogens of bacterial respiratory tract infections in livestock animals such as cattle and swine. The inventors have also found that a compound represented by the formula (IIIb) using josamycin as a lead scaffold has remarkably strong antibacterial activities against main pathogens of bacterial respiratory tract infections in livestock animals such as cattle and swine.

The inventors of the present invention produce the derivatives in accordance with the following method. That is, the above-mentioned compound as one of the derivatives is produced through the following series of steps of: (A) selectively epoxidizing a protected product modified with an appropriate protective group at the C-12 and C-13 olefm positions; (B) cleaving the epoxy moiety with an azide; (C) reducing the resultant azide product to afford an amino alcohol; (D) introducing a desired side chain by a Borch reductive amination reaction (Borch, R. F.et al. J. Org. Chem, (1972), 37, 1673.); and (E) finally performing deprotection.

This production method has problems such as a decrease in yield and a complicated operation in purification, which are caused by formation of a by-product having two side chains introduced in the Borch reductive amination reaction in the step (D). In general, in the case where an aldehyde to be introduced is bulky to some extent, the Borch reductive amination reaction preferentially gives a monoalkyl product owing to steric hindrance of the aldehyde. However, when the production method was employed for the above-mentioned compound, a dialkyl product was formed as a by-product because of high substrate dependency. In addition, picoline borane is known as a reducing agent capable of suppressing the formation of the dialkyl product as the by-product. However, in the Borch reduction into the above-mentioned compound with the reducing agent as well, preferential formation of the monoalkyl product was not observed.

Further, in methylation of an amino group, it is very difficult to obtain a monomethyl product, and a dimethyl product is generally formed. In actuality, when the methylation was employed for the above-mentioned compound, only the dimethyl product was formed. 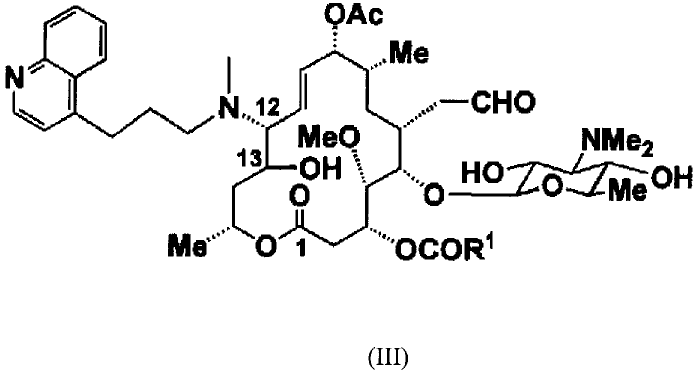 (IIIa): R¹ = Et, (IIIb): R¹ = Me

### SUMMARY OF INVENTION

### TECHNICAL PROBLEM

An object of the present invention is to provide a novel intermediate and a novel production method for a compound represented by the formula (III) or any other compound effective for the bacterial respiratory tract infections in animals described above via the intermediate easily in high yield.

That is, an object of the present invention is to provide a method of producing a compound represented by the formula (III) with ease in good yield while suppressing production of the above-mentioned by-products in a production step, and a monoalkyl product (I) that is an important intermediate for suppressing production of the by-products.

### SOLUTION TO PROBLEM

The inventors of the present invention have made intensive studies to avoid the problems described above. As a result, the inventors have solved the problems by avoiding reductive alkylation from an amino group, which leads to a decrease in yield. That is, the inventors have succeeded in producing a monoalkylamino intermediate (I) of interest by directly reducing an aza wittig-type intermediate formed by an azide group and a phosphine in an azide obtained by ring-opening of an epoxide. In particular, when a method of producing a compound represented by the formula (III) via an intermediate (II) having a monomethylated amino group is employed, it is possible to produce a compound of interest by using a minimum amount of an aldehyde corresponding to a desired side chain used in subsequent Borch reduction. As a result, the inventors have achieved a decrease in reagent amount, an improvement in yield, and facilitation of purification, and the present invention has been completed as an efficient and novel production method.

The present invention provides a method of efficiently producing a compound represented by the formula (III) exhibiting excellent antibacterial activities against bacterial infections in animals, and novel intermediates (I) and (II).

### ADVANTAGEOUS EFFECTS OF INVENTION

The monoalkylated amino product (I) obtained in the production method according to the present invention is an extremely useful synthetic intermediate. Specifically, when the compound represented by the formula (III) is synthesized via the monomethylated amino product (II), the reagent amount of an aldehyde corresponding to a side chain to be subsequently added can be reduced to an almost theoretical equivalent. Therefore, this has allowed a production method for the compound represented by the formula (III), which is inexpensive and facilitates purification, to be provided.

The compound represented by the formula (III) provided by the production method of the present invention is effective not only for Gram-positive bacteria, mycoplasmae, chlamydiae, and rickettsiae in the same manner as general macrolides but also for Gram-negative bacteria problematic in infections in animals. Further, the compound produced in the present invention can exhibit excellent antibacterial effects against pathogens of respiratory tract infections in animals such as cattle and swine. In addition, the use of the compound produced in the present invention allows infections in animals, such as lung infection, mastitis, bacteremia, septicemia, and diarrhea, to be treated or prevented effectively.

### DESCRIPTION OF EMBODIMENTS

The inventors of the present invention have succeeded for the first time in producing a compound represented by the formula (I) or a salt thereof, and have been able to find a novel method of producing an excellent animal antibacterial compound represented by the formula (III) in good yield via the compound represented by the formula (I).

That is, the present invention relates to, for example, the following items:
(1) a compound, which is represented by the following formula (I), or a pharmacologically acceptable salt thereof: 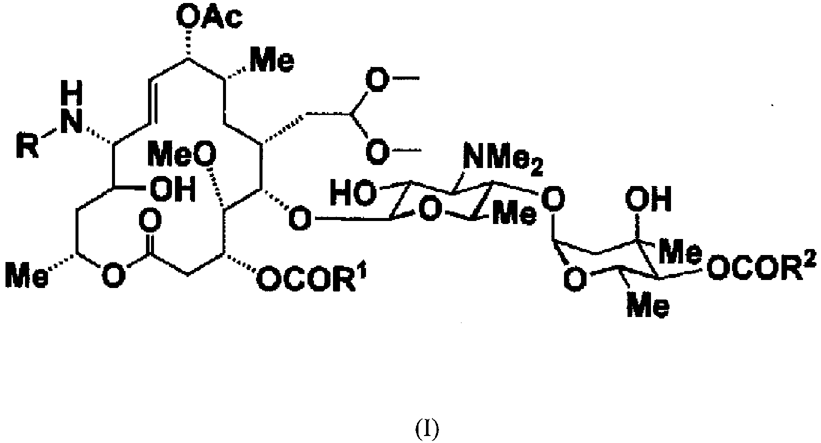 (where R represents a lower alkyl group, an alkyl group that may have a substituent, an aralkyl group that may have a substituent, or a heterocyclic alkyl group that may have a substituent, R¹ represents a methyl group or an ethyl group, and R² represents an ethyl group or an isobutyl group);
(2) a compound, which is represented by the following formula (II), or a pharmacologically acceptable salt thereof: 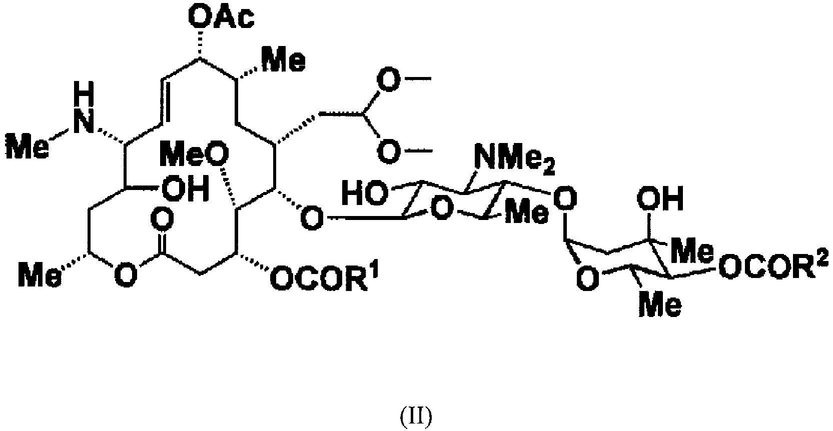 (where R¹ represents a methyl group or an ethyl group, and R² represents an ethyl group or an isobutyl group);
(3) a compound according to the above-mentioned item (2), in which R¹ and R² each represent an ethyl group; and
(4) a compound according to the above-mentioned item (2), in which R¹ represents a methyl group, and R² represents an isobutyl group.
   Further, as other embodiments, the present invention relates to the following items:
(5) a production method for a compound represented by the following formula (III), including allowing a compound represented by the formula (I) to react with 3-(quinolin-4-yl)propylaldehyde and subjecting the resultant to a reduction reaction: 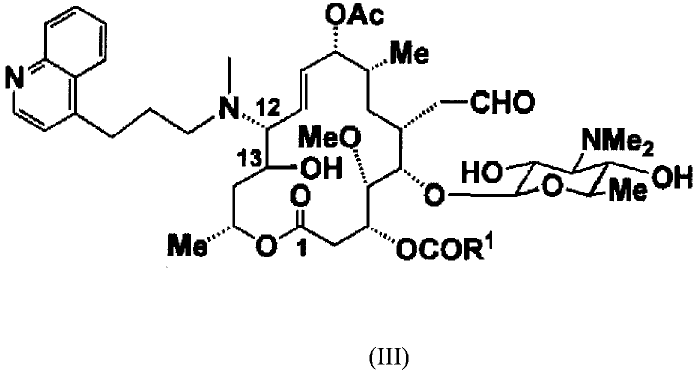 (where R¹ represents a methyl group or an ethyl group);
(6) a production method for the compound represented by the formula (I) as defined in the above-mentioned item (1), including allowing a compound represented by the formula (IV) to react with an organophosphorous reagent and subsequently with an aldehyde reagent and subjecting the resultant to a reduction reaction: 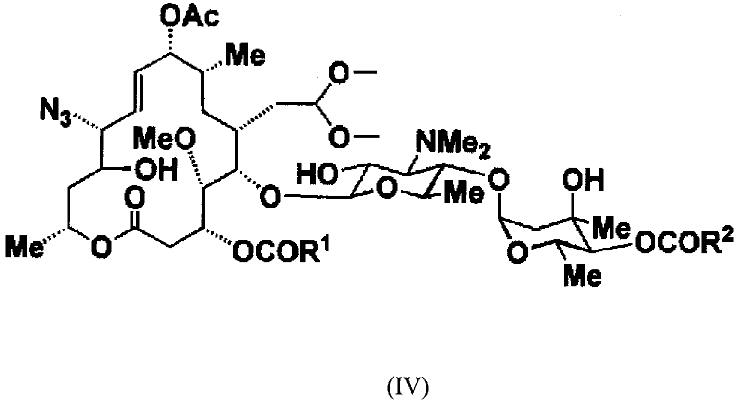 (where R¹ represents a methyl group or an ethyl group, and R² represents an ethyl group or an isobutyl group); and
(7) a production method for a compound represented by the formula (II) as defined in any one of claims 2 to 4, including allowing a compound represented by the formula (IV) to react with an organophosphorous reagent and subsequently with paraformaldehyde and subjecting the resultant to a reduction reaction.

The "lower alkyl group" as used herein refers to an alkyl group having 1 to 6 carbon atoms. The alkyl group may be any one of linear, branched, and cyclic alkyl groups. Examples thereof include: C₁₋₆ alkyl groups such as methyl, ethyl, propyl, isopropyl, butyl, isobutyl, sec-butyl, tert-butyl, pentyl, and hexyl; and C₃₋₆ cycloalkyl groups such as cyclopropyl, cyclobutyl, cyclopentyl, and cyclohexyl.

The "alkyl group that may have a substituent, aralkyl group that may have a substituent, or heterocyclic alkyl group that may have a substituent" as used herein specifically refers to a phenylalkyl group that may have a substituent, a naphthylalkyl group that may have a substituent, a quinolinylalkyl group that may have a substituent, an isoquinolinylalkyl group that may have a substituent, a quinolinylalkenyl group that may have a substituent, a pyridinylalkyl group that may have a substituent, an indolylalkyl group that may have a substituent, a pyrrolylalkyl group that may have a substituent, a thiophenylalkyl group that may have a substituent, an imidazolylalkyl group that may have a substituent, a purinylalkyl group that may have a substituent, a naphthyridinylalkyl group that may have a substituent, or the like. Examples thereof include a benzyl group, a 2-phenylethyl group, a 3-phenylpropyl group, a 3-phenylpropen-2-yl group, a 4-phenylbutyl group, a 5-phenylpentyl group, a 6-phenylhexyl group, a 7-phenylheptyl group, a 3-(4-methylphenyl)propyl group, a 3-(2-benzyloxyphenyl)propyl group, a 3-(4-methoxyphenyl)propyl group, a 3-(4-hydroxyphenyl)propyl group, a 3-(4-dimethylaminophenyl)propyl group, a 3-(4-nitrophenyl)propyl group, a 3-(4-fluorophenyl)propyl group, a 3-(4-trifluoromethyl)phenylpropyl group, a 3-(2-benzyloxyphenyl)propyl group, a 3,3-diphenylpropyl group, a 3-(4-biphenylyl)propyl group, a 3-(1-naphthyl)propyl group, a 3-(2-naphthyl)propyl group, a 3-(quinolin-3-yl)propyl group, a 3-(quinolin-4-yl)propyl group, a 3-(quinolin-6-yl)propyl group, a 3-(quinolin-7-yl)propyl group, a 3-(quinolin-4-yl)propen-2-yl group, a 4-(quinolin-4-yl)butyl group, a 3-(6-chloroquinolin-4-yl)propyl group, a 3-(6-methoxyquinolin-4-yl)propyl group, a 2-(N-methyl-N-(quinolin-4-yl)amino)ethyl group, a 3-(isoquinolin-4-yl)propyl group, a pyridin-4-ylmethyl group, a 3-(pyridin-3-yl)propyl group, a 3-(pyridin-4-yl)propyl group, a 3-(1-imidazolyl-4-phenyl)propyl group, a 3-(indolyl-3-yl)propyl group, a 3-(1-methyl-1H-indol-3-yl)propyl group, a 3-(1-methyl-1H-pyrrol-2-yl)propyl group, a 3-(5-(pyridin-2-yl)thiophen-2-yl)propyl group, a 3-(4-(pyridin-3-yl)-1H-imidazolyl)propyl group, a 3-(4-(pyridin-4-yl)-1H-imidazolyl)propyl group, a 3-(theophyllin-7-yl)propyl group, a 3-(6-chloro-9H-purin-9-yl)propyl group, a 2-(7-chloro-1,8-naphthyridin-2(1H)-on-3-ylethyl group, a 6-(4-nitrophenyl)hexyl group, a 6-(4-fluorophenyl)hexyl group, a 1-benzylpiperidin-4-ylmethyl group, and a 3-(imidazo[5,1-b]thiazol-2-yl)propen-2-yl group.

Examples of the "organophosphorus reagent" as used herein include triphenylphosphine, trimethylphosphine, triethylphosphine, and tributylphosphine.

The compound represented by the formula (III) produced according to the present invention is effective for, for example, the following bacterial pathogens: as bacterial pathogens for swine diseases, *Bacillus anthracis, Brucella suis, Clostridium chauvoei, Leptospira, Salmonella serovar Dublin, S. Enteritidis, S. Typhimurium, S. Choleraesuis, Francisella tularensis, F. holarctia, F. mediasiatica, F. novicida, Bordetella bronchiseptica,* and toxigenic *Pasteurella multocida, Erysipelothrix rhusiopathiae, Brachyspira hyodysenteriae, Staphylococcus hyicus, Lawsonia intracellularis,* verocytotoxigenic *Escherichia coli* (VTEC), *Actinobacillus equuli, A. pleuropneumoniae, A. susi, Arcanobacterium pyogenes, Clostridium perfringens* type C, *Actinobacillus pleuropneumoniae, Mycobacterium avium*-intracellulare complex, enterotoxigenic *Escherichia coli* (ETEC), attaching and effacing *Escherichia coli* (AEEC), *Pasteurella multocida* types B and E, *Mycoplasma hyopneumoniae, Streptococcus suis, Haemophilus parasuis;* as bacterial pathogens for bovine diseases, *Bacillus anthracis, Brucella abortus, B. acanis, Mycobacterium bovis, Mycobacterium avium* subsp. *paratuberculosis, Clostridium chauvoei, Clostridium tetani, Leptospira, Salmonella serovar Dublin, S. Enteritidis, S. Typhimurium, S. Choleraesuis, Campylobacter fetus* subsp. *venerealis, C. fetus* subsp. *fetus, Clostridium septicum, C. sordellii, C. perfringens* (type A), *C. novyi* (type A), *Actinobacillus lignieresii, Clostridium perfringens, Corynebacterium renale, C. pilosum, C cystitidis,* enterotoxigenic *Escherichia coli* (ETEC), verocytotoxigenic *Escherichia coli* (VTEC), attaching and effacing *Escherichia coli* (AEEC), *Pasteurella multocida, Mannheimia haemolytica, P. trehalosi, Mycoplasma mycoides* subsp. *mycoides SC* (small colony) type, toxigenic bacteria *Clostridium botulinum* types C and D, *Mycoplasma bovis, M. bovigenitalium, M. dispar, Ureaplasma diversum, Mycoplasma alkalescens, M. arginini, M. bovigenitalium, M. bovirhinis, M. bovis, M. californicum, M. canadense, Fusobacterium necrophorum, Moraxella bovis, Histophilus somni, Actinomyces bovis, Listeria monocytogenes, Dermatophilus congolensis;* and as bacterial pathogens for poultry diseases, *Pasteulella multocida, Salmonella Pullorum (Salmonella enterica* subsp. *enterica* serovar *Gallinarum* biovar *Pullorum*) (pullorum disease), *Salmonella Gallinarum* (*Salmonella enterica* subsp. *enterica* serovar *Gallinarum* biovar *Gallinarum*)(fowl typhoid), *S. enterica, S. Typhimurium, S. Avibacterium, Haemophilus paragallinarum, Clostridium perfringens* types A and C, *Escherichia coli, Staphylococcus aureus, S. hyicus,* and toxigenic bacteria *Clostridium botulinum* type C.

In addition, the compound represented by the formula (III) produced according to the present invention is effective for, for example, the following bacterial diseases problematic in infections in animals in particular. In the case of swine, the compound is effective for, for example, swine anthrax, swine brucellosis, swine blackleg, swine leptospirosis, swine Weil's disease, swine salmonellosis, swine tularemia, atrophic rhinitis in swine, swine erysipelas, swine exudative epidermitis (exudative dermatitis, *Staphylococcus hyicus* infection), swine proliferative enteritis, swine edema disease, swine actinobacillosis, *Arcanobacterium pyogenes* infection in swine, swine necrotic enteritis, swine pleuropneumonia, mycobacterial infection in swine, swine colibacillosis, swine hemorrhagic septicemia, swine pasteurellosis (pneumonic pasteurellosis), streptococcal infection in swine, *Haemophilus parasuis* infection in swine (Glasser's disease), *Actinomyces pyogenes* infection in swine, swine yersiniosis, *Bacteroides* infection in swine, swine brucellosis, swine dysentery, swine enterotoxemia, streptococcal infection in swine, swine staphylococcosis, *Pseudomonas aeruginosa* infection in swine, swine eperhythrozoonosis, chlamydial infection in swine, mycoplasmal pneumonia in swine, and swine mycoplasmal arthritis. In the case of cattle, the compound is effective for, for example, bovine anthrax, bovine brucellosis, bovine tuberculosis, bovine Johne's disease, bovine blackleg, bovine tetanus, leptospirosis (bovine leptospirosis), bovine nocardiosis, bovine enterotoxemia, bovine tuberculosis, salmonellosis (bovine salmonellosis), bovine genital campylobacteriosis, bovine malignent edema, bovine actinobacillosis, bovine necrotic enteritis, *Corynebacterium* urinary tract infection in cattle, bovine colibacillosis, bovine hemorrhagic septicemia, *Pasteurella* (*Mannheimia*) infection in cattle, mastitis, coliform mastitis, *Haemophilus somnus* infection in cattle, contagious bovine pleuropneumonia, bovine botulism, bovine mycoplasmal pneumonia, bovine mycoplasmal mastitis, bovine liver abscess, infectious bovine keratoconjunctivitis, bovine cystitis, bovine pyelonephritis, bovine listeriosis, bovine necrobacillosis, bovine actinomycosis, bovine dermatophilosis, contagious bovine pleuropneumonia, abortion and infertility in cattle, sporadic bovine encephalomyelitis, bovine polyarthritis, tick fever, ehrlichiosis, bovine petechial fever, coxiellosis, anaplasmosis, and bovine eperhythrozoonosis. In the case of equine diseases, the compound is effective for equine glanders, equine melioidosis, equine tetanus, equine paratyphoid, equine *Klebsiella* infection, contagious equine metritis, *Rhodococcus equi* infection, equine strangles, chlamydial infection in horses, and Potomac horse fever. In the case of ovine and caprine diseases, the compound is effective for, for example, brucellosis, ovine dysentery, pseudotuberculosis, nonsuppurative polyarthritis, *Erysipelothrix rhusiopathiae* infection in sheep, clostridial infection in sheep, contagious ovine digital dermatitis, tularemia, heartwater, contagious ophthalmia, enzootic abortion in ewes, ovine polyarthritis, transmissible serositis, contagious agalactia, and contagious caprine pleuropneumonia. In the case of dogs and cats, the compound is effective for canine leptospirosis, canine Lyme disease, canine brucellosis, canine campylobacteriosis, *Bordetella* infection in dogs, anaerobic infection in dogs and cats, feline leptospirosis, feline tuberculosis, canine ehrlichiosis, salmon poisoning, cat-scratch disease, feline hemobartonellosis, chlamydial infection in cats, and mycoplasmosis in dogs and cats. In the case of poultry, the compound is effective for, for example, fowl cholera, anatipestifer infection in birds, avian paratyphoid, *Salmonella arizonae* infection, avian staphylococcosis, *Histophilus somni* infection, actinomycosis, listeriosis, dermatophilosis, digital papillomatosis, avian pasteurellosis, *Salmonella* infection in poultry, salmonellosis (avian paratyphoid), avian tuberculosis, infectious coryza, *Erysipelothrix rhusiopathiae* infection in birds, avian campylobacteriosis, clostridial disease in poultry, avian necrotic enteritis, avian colibacillosis, avian staphylococcosis, avian botulism, streptococcal infection in birds, avian spirochetosis, avain rhinotracheitis, aegyptianellosis, avian chlamydiosis, avian mycoplasmosis, and mycoplasmal synovitis in poultry. In the case of fish, the compound is effective for, for example, vibriosis, non-motile aeromonas infection, motile aeromonad septicemia, bacterial kidney disease, edwardsiellosis, coldwater disease, columnaris disease, pseudotuberculosis, pasteurellosis, red mouth disease, nocardiosis, mycobacterial infection in fish, *Pseudomonas* infection, bacterial gill disease, and streptococcal infection.

Further, the compound may be used as not only a therapeutic drug for the diseases exemplified above but also a therapeutic drug for other diseases based on effects peculiar to a macrolide except antibacterial actions, such as an immunostimulatory action and and an effect on a biofilm.

The production methods for the compounds represented by the formula (I) and the formula (II) or salts thereof and the compound represented by the formula (III) of the present invention are described below. Herein, the production methods are described with reference to compounds (Ia), (IIa), (IIIa), (IVa), and (Va) represented by the formulae where R represents a methyl group, and R¹ and R² each represent an ethyl group and compounds (Ib), (IIb), (IIIb), (IVb), and (Vb) represented by the formulae where R¹ represents a methyl group, and R² represents an isobutyl group. The present invention is not limited to the following step chart, and the compounds can be produced by a method in conformity to the chart.

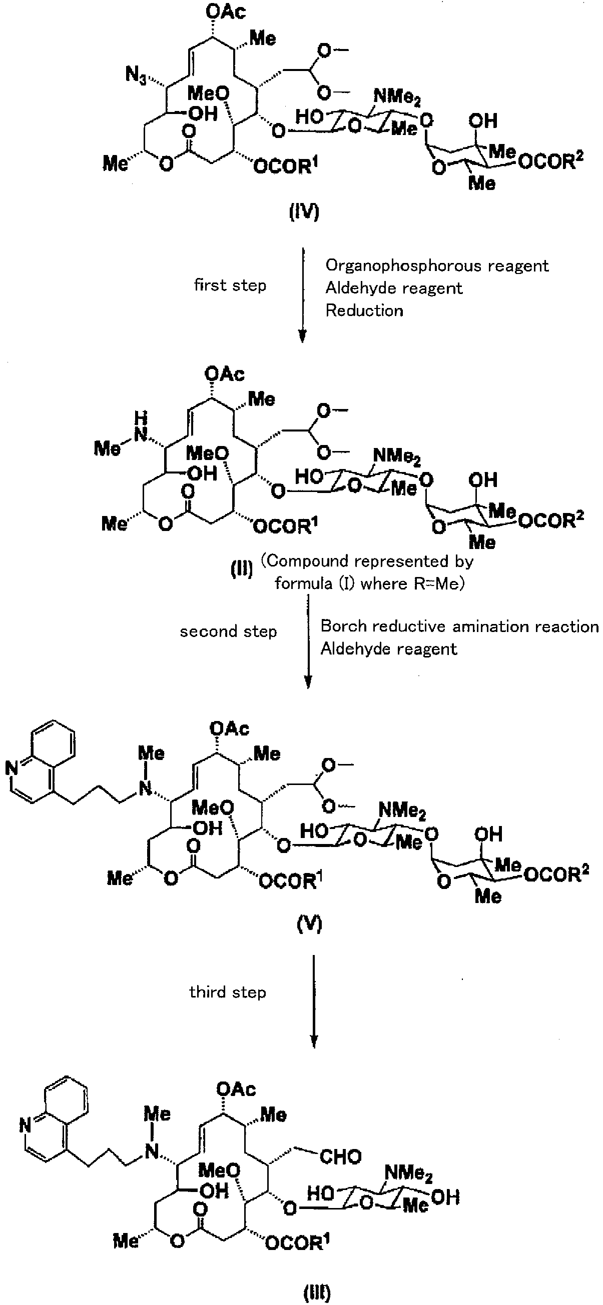

### First step

To 9-O-acetyl-12-azido-12,13-dihydro-13-hidroxymidecamycin 18-dimethylacetal (Example 3 in WO 2002/064607 A1: (IVa)) or 9-O-acetyl-12-azido-12,13-dihydro-13-hydroxyjosamycin 18-dimethylacetal (IVb) was added an organophosphorous reagent, and disappearance of the raw materials was confirmed, followed by addition of a desired aldehyde reagent. The resultant was allowed to react for several hours to form a non-isolable intermediate, and the intermediate was reduced to afford a monoalkylated amino product (I) of interest.

The monoalkyl group in this step is a side chain of interest. For example, a method involving introducing a 3-(quinolin-4-yl)propyl group and methylating the group may be employed without any problem. However, the reaction is very efficiently carried out by performing the monomethylation as the first step because the reaction can be completed with a required amount (1 to 1.5 equivalents) of a reagent for the subsequent introduction of the side chain. Therefore, in this description, a production method involving performing, for example, 3-(quinolin-4-yl)propylation via a monomethylated amino product is described.

It should be noted that the alkylation of an amino group performed after the monomethylation is not limited to the 3-(quinolin-4-yl)propylation, and an appropriate aldehyde may be selected and used depending on the intended use.

As the aldehyde reagent to be used in the monoalkylation reaction, when a lower alkyl group such as a methyl group, an ethyl group, or a butyl group is intended to be introduced in the alkylation, there may be used paraformaldehyde, acetaldehyde, propionaldehyde, or the like, and when an alkyl group that may have a substituent, an aralkyl group that may have a substituent, a heterocyclic alkyl group that may have a substituent, or the like, such as a 3-(quinolin-4-yl)propyl group, is intended to be introduced in the alkylation, there may be used an aldehyde corresponding to each of the groups.

Examples of the "aldehyde reagent" as used herein include paraformaldehyde, acetaldehyde, propionaldehyde, benzaldehyde, 2-phenylethylaldehyde, 3-phenylpropylaldehyde, cinnamylaldehyde, 4-phenylbutyraldehyde, 5-phenylpentylaldehyde, 6-phenylhexylaldehyde, 7-phenylheptylaldehyde, 3-(4-methylphenyl)propylaldehyde, 3-(2-benzyloxyphenyl)propylaldehyde, 3-(4-methoxyphenyl)propylaldehyde, 3-(4-hydroxyphenyl)propylaldehyde, 3-(4-dimethylaminophenyl)propylaldehyde, 3-(4-nitrophenyl)propylaldehyde, 3-(4-fluorophenyl)propylaldehyde, 3-(4-trifluoromethyl)phenylpropylaldehyde, 3-(2-benzyloxyphenyl)propylaldehyde, 3,3-diphenylpropylaldehyde, 3-(4-biphenylyl)propylaldehyde, 3-(1-naphthyl)propylaldehyde, 3-(2-naphthyl)propylaldehyde, 3-(quinolin-3-yl)propylaldehyde, 3-(quinolin-4-yl)propylaldehyde, 3-(quinolin-6-yl)propylaldehyde, 3-(quinolin-7-yl)propylaldehyde, 3-(quinolin-4-yl)acrylaldehyde, 4-(quinolin-4-yl)butyraldehyde, 3-(6-chloroquinolin-4-yl)propylaldehyde, 3-(6-methoxyquinolin-4-yl)propylaldehyde, 2-(N-methyl-N-(quinolin-4-yl)amino)ethylaldehyde, 3-(isoquinolin-4-yl)propylaldehyde, 4-pyridinecarbaldehyde, 3-(pyridin-3-ylpropylaldehyde, 3-(pyridin-4-yl)propylaldehyde, 3-(1-imidazolyl-4-phenyl)propylaldehyde, 3-(indol-3-yl)propylaldehyde, 3-(1-methyl-1H-indol-3-yl)propylaldehyde, 3-(1-methyl-1H-pyrrol-2-yl)propylaldehyde, 3-(5-(pyridin-2-yl)thiophen-2-yl)propylaldehyde, 3-(4-(pyridin-3-yl)-1H-imidazo lyl)propylaldehyde, 3-(4-(pyridin-4-yl)-1H-imidazolyl)propylaldehyde, 3-(theophyllin-7-yl)propylaldehyde, 3-(6-chloro-9H-purin-9-yl)propylaldehyde, 2-(7-chloro-1,8-naphthyridin-2(1H)-on-3-ylethylaldehyde, 6-(4-nitrophenyl)hexylaldehyde, 6-(4-fluorophenyl)hexylaldehyde, 1-benzylpiperidin-4-ylaldehyde, and 3-(imidazo[5,1-b]thiazol-2-yl)propen-2-ylaldehyde.

The aldehyde reagent used for the monomethylation reaction is preferably paraformaldehyde, and is recommended to be added after confirmation of an active intermediate formed with a phosphine as described below (disappearance of raw materials).

A solvent used in this reaction may be, for example, chloroform or methylene chloride, and an organophosphorous reagent used may be, for example, trimethylphosphine, triphenylphosphine, triethylphosphine, or tributylphosphine. It is preferably recommended to use 1 to 3 equivalents of trimethylphosphine. This reaction proceeds in good yield in the range of 0°C to 80°C, and the reaction time is 1 hour to 36 hours. After confirmation of disappearance of the raw materials, 2 to 10 equivalents of paraformaldehyde were added. The reaction proceeds in the range of 0°C to 80°C, and the reaction time is 1 hour to 36 hours.

Subsequently, in the reduction reaction, it is recommended to add a lower alcohol such as methanol or ethanol having dissolved therein 1 to 5 equivalents of sodium borohydride to the reaction solution described above. The reaction proceeds in good yield in the range of 0°C to 50°C, and the reaction time is 5 minutes to 1 hour.

A reducing agent used in this reduction reaction may be sodium borohydride or any other reducing agent such as picoline borane or pyridine borane, and is preferably sodium borohydride.

The compounds represented by the formula (I) and the formula (II) may each be in the form of a salt. The salt is preferably a pharmacologically acceptable salt.

An acid addition salt is typically exemplified by, but not limited to: inorganic acid salts such as a hydrochloric acid salt, a sulfuric acid salt, a nitric acid salt, a hydrobromic acid salt, a hydroiodic acid salt, and a phosphoric acid salt; organic carboxylic acid salts such as an acetic acid salt, a trifluoroacetic acid salt, a lactic acid salt, a citric acid salt, an oxalic acid salt, a succinic acid salt, a glutaric acid salt, a malic acid salt, a tartaric acid salt, a fumaric acid salt, a mandelic acid salt, a maleic acid salt, a benzoic acid salt, a nicotinic acid salt, and a phthalic acid salt; organic sulfonic acid salts such as a methanesulfonic acid salt, an ethanesulfonic acid salt, a 2-hydroxyethanesulfonic acid salt, a benzenesulfonic acid salt, a p-toluenesulfonic acid salt, a 2-naphthalenesulfonic acid salt, and a camphorsulfonic acid salt; and acidic amino acid salts such as an aspartic acid salt and a glutamic acid salt. Preferred examples of the acid addition salt include salts with inorganic acids such as hydrochloric acid, hydrobromic acid, sulfuric acid, and phosphoric acid and salts with with organic acids such as oxalic acid, maleic acid, methanesulfonic acid, p-toluenesulfonic acid, and citric acid. More preferred examples of the acid addition salt include salts with hydrochloric acid, hydrobromic acid, sulfuric acid, and methanesulfonic acid.

Further, the compounds represented by the formula (I) and the formula (II) of the present invention may each be used in the form of a solvate. A solvent for the solvate is exemplified by, but not limited to, water, methanol, ethanol, ethylene glycol, propylene glycol, ethyl acetate, and butyl acetate. Preferred examples of the solvent for the solvate include water, ethanol, and ethyl acetate.

### Second step

To the monomethylamino product (II) obtained in the reaction described above (compound represented by the formula (I) where R represents a methyl group) was added 3-(quinolin-4-yl)propylaldehyde in the presence of acetic acid, followed by Borch reductive amination reaction with sodium cyanoborohydride, to thereby afford a compound (V) of interest. An aldehyde reagent in this reaction may be used in 1 to 1.5 equivalents, and a solvent may be a lower alcohol such as methanol or ethanol or any other solvent such as acetonitrile or methylene chloride.

Acetic acid to be added may be used in 1 to 5 equivalents, and a reducing agent in the Borch reductive amination reaction may be sodium cyanoborohydride or any other reducing agent such as sodium acetoxyborohydride or picoline borane. The reducing agent is preferably sodium cyanoborohydride or the like. It is recommended to use 1 to 5 equivalents of the reducing agent. The reaction proceeds in good yield in the range of 0°C to 50°C, and the reaction time is 5 minutes to 24 hours.

The aldehyde reagent to be used in the second step is the same as that described above.

### Third step

The compound (V) described above and modified with an amino group was allowed to react with difluoroacetic acid in a mixed solvent of acetonitrile and water to remove a protective group at the C-18 position and neutral sugars, to thereby afford a compound represented by the formula (III), specifically the formula (IIIa) or the formula (IIIb). It is recommended to use, as a solvent used in this reaction, a 10-fold amount (g/ml) to a 300-fold amount (g/ml) of a mixed solution of equal amounts of acetonitrile and water. Further, in addition to difluoroacetic acid, monofluoroacetic acid, trifluoroacetic acid, acetic acid, or the like may be used, and is recommended to be used in 1 to 30 equivalents. The reaction proceeds in good yield in the range of 20°C to 50°C, and the reaction time is 12 hours to 4 days.

Hereinafter, the present invention is specifically described by way of Examples. It should be noted that the present invention is by no means limited to Examples, and encompasses all of synthesis, production, extraction, and purification methods for the compounds by known means based on the properties of the compounds revealed by the present invention as well as modification means of Examples.

### EXAMPLE 1

### Production method for 9-O-acetyl-12,13-dihydro-13-hydroxy-12-(N-methyl-N-(3-(quinolin-4-yl)propyl)aminomidecamycin 18-dimethylacetal ((Va); compound represented by the formula (V) where R¹=R²=an ethyl group)

961 mg of 9-O-acetyl-12-azido-12,13-dihydro-13-hydroxymidecamycin 18-dimethylacetal, a compound obtained in accordance with a method disclosed in the patent literature (Example 3 in WO 2002/064607 A1), were dissolved by adding 24 ml of chloroform, and 2 ml of a 1 M solution of trimethylphosphine in toluene were added. The mixture was stirred at room temperature for 1 hour. 150 mg of paraformaldehyde were added, and the mixture was stirred for an additional 5 hours. Then, 189 mg of sodium borohydride were dissolved in 1 ml of methanol, and the resultant was gradually added. The mixture was stirred at room temperature for 30 minutes and concentrated under reduced pressure, and the resultant residue was purified by silica gel column chromatography (chloroform-methanol-aqueous ammonia (200:10:1)) to afford 630 mg of a compound (IIa).

### Physicochemical properties of this compound

(1) Mass spectrum (ESI): m/z 949 (M+H)⁺
(2) ¹H NMR spectrum (400 MHz, CDCl₃) δ (ppm): 0.98 (d, 3H), 1.12 (s, 3H), 1.12 (d, 3H), 1.14 (t, 3H), 1.18 (t, 3H), 1.28 (d, 3H), 1.31 (d, 3H), 1.40-1.80 (m, 5H), 1.85 (dd, 1H), 2.07 (s, 3H), 2.18 (d, 2H), 2.30-2.55 (m, 10H), 2.43 (s, 3H), 2.51 (s, 6H), 2.74 (dd, 1H), 2.86 (dd, 1H), 3.20 (s, 3H), 3.28 (s, 3H), 3.45 (br d, 3H), 3.53 (s, 3H), 3.58 (dd, 1H), 4.02 (br s, 1H), 4.47 (dq, 1H), 4.52 (m, 1H), 4.63 (t, 1H), 4.68 (d, 1H), 5.08 (d, 1H), 5.18 (br d, 1H), 5.35 (dd, 9-H), 5.73 (dd, 1H), 5.95 (dd, 1H).

630 mg of the compound (IIa) obtained as described above were dissolved by adding 35 ml of methanol, and 203 mg of 3-(quinolin-4-yl)propylaldehyde and 229 µl of acetic acid were added. The mixture was stirred at room temperature for 20 minutes. To the reaction solution were added 126 mg of sodium cyanoborohydride, and the mixture was stirred for an additional 1 hour. Then, to the reaction solution were added 30 ml of saturated aqueous sodium bicarbonate, and the resultant was extracted twice with 30 ml of chloroform. The organic layer was washed successively with 50 ml of saturated aqueous sodium bicarbonate and 50 ml of brine. The resultant organic layer was dried over anhydrous sodium sulfate and then filtered. The filtrate was concentrated under reduced pressure. The resultant residue was purified by silica gel column chromatography (chloroform-methanol-aqueous ammonia (200:10:1)) to afford 654 mg of the title compound.

### Physicochemical properties of this compound

(1) Mass spectrum (FAB): m/z 1,118 (M+H)⁺
(2) ¹H NMR spectrum (300 MHz, CDCl₃ δ (ppm): 0.92 (d, 3H), 1.06 (t, 3H), 1.11 (s, 3H), 1.17 (t, 3H), 1.28 (d, 1H), 1.28 (d, 1H), 1.51 (br t, 1H), 1.51 (br dd, 1H), 1.83 (dd, 1H), 2.02 (d, 1H), 2.05 (s, 3H), 2.35 (s, 3H), 2.51 (s, 6H), 2.65 (m, 2H), 2.74 (dd, 1H), 3.17 (s, 3H), 3.27 (s, 3H), 3.32 (m, 1H), 3.32 (m, 1H), 3.43 (br d, 1H), 3.53 (s, 3H), 3.59 (dd, 1H), 3.98 (br d, 1H), 3.98 (br dd, 1H), 4.48 (dq, 1H), 4.54 (d, 1H), 4.58 (t, 1H), 4.61 (d, 1H), 5.00 (ddq, 1H), 5.07 (d, 1H), 5.16 (br dd, 1H), 5.38 (br s, 1H), 5.72 (br d, 1 H), 5.81 (br d, 1H), 7.24 (d, 1H), 7.56 (ddd, 1H), 7.69 (ddd, 1H), 8.03 (br d, 1H), 8.09 (br d, 1H), 8.78 (d, 1H).

### EXAMPLE 2

### Production method for 9-O-acetyl-4'-demycarosyl-12,13-dihydro-13-hydroxy-12-(N-methyl -N-(3-(quinolin-4-yl)propyl)aminomidecamycin (compound represented by the formula (IIIa))

60 mg of the compound of Example 1 were dissolved by adding 1 ml of acetonitrile, and 1 ml of water was added. 70 µl of difluoroacetic acid were added, and the mixture was stirred at 40°C for 60 hours. To the reaction solution were added 20 ml of saturated aqueous sodium bicarbonate, and the resultant was extracted with 40 ml of ethyl acetate. The organic layer was washed successively with 20 ml of saturated aqueous sodium bicarbonate and 20 ml of brine. The resultant organic layer was dried over anhydrous sodium sulfate and then filtered. The filtrate was concentrated under reduced pressure. The resultant residue was purified by preparative TLC (chloroform-methanol-aqueous ammonia (100:10:1)) to afford 24 mg of the title compound.

### Physicochemical properties of this compound

(1) Mass spectrum (FAB): m/z 872 (M+H)⁺
(2) ¹H NMR spectrum (300 MHz, CDCl₃ δ (ppm): 0.88 (d, 3H), 1.05 (br t, 3H), 1.12 (t, 3H), 1.21 (d, 3H), 1.22 (d, 3H), 1.50 (dt, 3H), 1.55 (br dd, 1H), 2.02 (m, 2H), 2.04 (s, 3H), 2.32 (s, 3H), 2.34 (t, 1H), 2.50 (s, 6H), 2.99 (dd, 1H), 3.06 (t, 1H), 3.27 (dq, 1H), 3.39 (br d, 1H), 3.48 (dd, 1H), 3.54 (s, 3H), 3.97 (br d, 1H), 4.02 (br dd, 1H), 4.45 (d, 1H), 5.00 (ddq, 1H), 5.17 (br s, 1H), 5.26 (br t, 1H), 5.76 (dd, 1H), 5.82 (br d, 1H), 7.23 (d, 1H), 7.54 (dd, 1H), 7.68 (dd, 1H), 8.04 (d, 1H), 8.09 (d, 1H), 8.78 (d, 1H), 9.67 (s, 1H).

### EXAMPLE 3

### Production method for 9-O-acetyl-12-azido-12,13-dihydro-13-hydroxyjosamycin 18-dimethylacetal (compound represented by the formula (IVb))

1.0 g of josamycin was dissolved in 15 µl of methylene chloride. To the solution were added 178 µl of pyridine, followed by gradual dropwise addition of 114 µl of acetyl chloride. The mixture was stirred at room temperature for 3 hours, and saturated aqueous sodium bicarbonate was then added. 35 ml of methylene chloride were added, and the resultant was washed successively with saturated aqueous sodium bicarbonate and brine. The organic layer was dried over anhydrous sodium sulfate and then filtered. The filtrate was evaporated under reduced pressure. The resultant concentrate was dissolved by adding 3 ml of methanol. To the solution were added 3 ml of methyl orthoformate and 322 mg of PPTS, and the mixture was stirred at 50°C for 33 hours. To the reaction solution was added saturated aqueous sodium bicarbonate, and the mixture was concentrated under reduced pressure and extracted twice with 50 ml of chloroform. The organic layer was washed successively with saturated aqueous sodium bicarbonate and brine. The organic layer was dried over anhydrous sodium sulfate and then filtered.

The filtrate was concentrated under reduced pressure. The resultant reaction product was dissolved by adding 12 ml of chloroform. To the solution were added 982 mg of 3-chloroperbenzoic acid, and the mixture was subjected to a reaction at room temperature for 14 hours. To the reaction solution were added 50 ml of ethanol, followed by gradual dropwise addition of 18 ml of a 5% sodium dithionite aqueous solution under cooling with ice. The mixture was stirred for 1 hour and then concentrated under reduced pressure. To the residue was added saturated aqueous sodium bicarbonate, and the resultant was extracted twice with 50 ml of chloroform. The organic layer was washed successively with 100 ml of saturated aqueous sodium bicarbonate and 100 ml of brine. The organic layer was dried over anhydrous sodium sulfate and then filtered. The filtrate was concentrated under reduced pressure. The resultant residue was passed through silica gel column chromatography (chloroform-methanol (50:1)) to afford crude 9-O-acetyl-12,13-dihydro-13-hydroxy-epoxyjosamycin 18-dimethylacetal.

280 mg of the compound obtained as described above were dissolved by adding 7.0 ml of ethanol-water (8:1) to the compound. To the solution were added 30 mg of ammonium chloride and 70 mg of sodium azide, and the mixture was stirred at 80°C for 24 hours. To the reaction solution were added 10 ml of water, and the mixture was concentrated under reduced pressure. Then, to the residue were added 50 ml of water, and the resultant was extracted with 100 ml of chloroform. The organic layer was washed successively with saturated aqueous sodium bicarbonate and brine. The organic layer was dried over anhydrous sodium sulfate and then filtered. The filtrate was concentrated under reduced pressure. The resultant residue was purified by silica gel column chromatography (chloroform-methanol-aqueous ammonia (200:10:1)) to afford 560 mg of the title compound.

### Physicochemical properties of this compound

(1) Mass spectrum (ESI): m/z 974 (M+H)⁺
(2) ¹H NMR spectrum (400 MHz, CDCl₃ δ (ppm): 0.94 (d, 3-H), 0.96 (d, 3-H), 1.12 (s, 3H), 1.13 (d, 3H), 1.30 (d, 3H), 1.50-1.90 (m, 3H), 2.04 (s, 3H), 2.10 (s, 3H), 2.50 (br s, 6H), 2.61 (dd, 1-H), 2.85 (dd, 1H), 3.16 (s, 3H), 3.27 (s, 3H), 3.53 (s, 3H), 3.57 (dd, 2'-H), 3.93 (br dd, 1H), 4.00 (br d, 1H), 4.25 (br s, 1H), 4.40-4.65 (m, 4H), 5.05-5.15 (m, 3H), 5.32 (br s, 1H), 5.78 (br s, 2H).

### EXAMPLE 4

### Production method for 9-O-acetyl-12,13-dihydro-13-hydroxy-12-(N-methyl-N-(3-(quinolin-4-yl)propyl)aminojosamycin 18-dimethylacetal ((Vb); compound represented by the form ula (V) where R¹=a methyl group and R²=an isobutyl group)

500 mg of the compound of Example 3 were dissolved by adding 12 ml of chloroform, and 1 ml of a 1 M solution of trimethylphosphine in toluene was added. The mixture was stirred at room temperature for 1 hour. 80 mg of paraformaldehyde were added, and the mixture was stirred for an additional 5 hours. 100 mg of sodium borohydride were dissolved in 500 µl of methanol, and the solution was gradually added. The mixture was stirred at room temperature for 30 minutes and concentrated under reduced pressure, and the resultant residue was purified by silica gel column chromatography (chloroform-methanol-aqueous ammonia (200:10:1)) to afford 315 mg of a compound (IIb).

### Physicochemical properties of this compound

(1) Mass spectrum (ESI): m/z 963 (M+H)⁺
(2) ¹H NMR spectrum (400 MHz, CDCl₃) δ (ppm): 0.96 (d, 3H), 1.10 (s, 3H), 1.12 (d, 3H), 1.20-1.30 (m, 6H), 1.40-1.80 (m, 5H), 2.03 (s, 3H), 2.10 (s, 3H), 2.28 (d, 2H), 2.43 (s, 3H), 2.49 (s, 6H), 2.66 (dd, 1H), 2.80 (dd, 1H), 3.23 (s, 3H), 3.28 (s, 3H), 3.52 (s, 3H), 3.56 (dd, 1H), 3.86 (br s, 1H), 4.00 (br d, 1H), 4.45 (dq, 1H), 4.52 (d, 1H), 4.60 (d, 1H), 5.07 (d, 1H), 5.15 (m, 2H), 5.31 (dd, 9-H), 5.65 (dd, 1H), 5.80 (dd, 1H).

315 mg of the compound (IIb) described above were dissolved by adding 20 ml of methanol, and 105 mg of 3-(quinolin-4-yl)propylaldehyde and 120 µl of acetic acid were added. The mixture was stirred at room temperature for 20 minutes. To the reaction solution were added 65 mg of sodium cyanoborohydride, and the mixture was stirred for an additional one hour. Subsequently, to the reaction solution were added 20 ml of saturated aqueous sodium bicarbonate, and the resultant was extracted twice with 20 ml of chloroform. The organic layer was washed successively with 40 ml of saturated aqueous sodium bicarbonate and 40 ml of brine. The organic layer was dried over anhydrous sodium sulfate and then filtered. The filtrate was concentrated under reduced pressure. The resultant residue was purified by silica gel column chromatography (chloroform-methanol-aqueous ammonia (200:10:1)) to afford 320 mg of the title compound.

### Physicochemical properties of this compound

(1) Mass spectrum (ESI): m/z 1,132 (M+H)⁺
(2) ¹H NMR spectrum (400 MHz, CDCl₃ δ (ppm): 0.92 (d, 3H), 0.95 (d, 3H), 1.11 (s, 3H), 1.12 (d, 3H), 1.15 (d, 3H), 1.26 (d, 3H), 2.03 (s, 3H), 2.05 (s, 3H), 2.22 (s, 3H), 2.27 (d, 1H), 2.50 (s, 6H), 2.65 (m, 2H), 3.15 (s, 3H), 3.27 (s, 3H), 3.53 (s, 3H), 3.57 (dd, 1H), 3.96 (m, 2H), 4.50 (m, 3H), 4.60 (d, 1H), 5.00 (m, 1H), 5.06 (d, 1H), 5.15 (br s, 1H), 5.37 (br s, 1H), 5.72 (br dd, 1H), 5.81 (br d, 1H), 7.23 (d, 1H), 7.55 (dd, 1H), 7.68 (dd, 1H), 8.02 (d, 1H), 8.08 (d, 1H), 8.78 (d, 1H).

### EXAMPLE 5

### Production method for 9-O-acetyl-4'-demycarosyl-12,13-dihydro-13-hydroxy-12-(N-methyl -N-(3-(quinolin-4-yl)propyl)aminojosamycin (compound represented by the formula (III b))

66 mg of the compound of Example 4 were dissolved by adding 1 ml of acetonitrile, and 1 ml of water was added. 73 µl of difluoroacetic acid were added, and the mixture was stirred at 40°C for 60 hours. To the reaction solution were added 20 ml of saturated aqueous sodium bicarbonate, and the resultant was extracted with 40 ml of ethyl acetate. The organic layer was washed successively with 20 ml of saturated aqueous sodium bicarbonate and 20 ml of brine. The organic layer was dried over anhydrous sodium sulfate and then filtered. The filtrate was concentrated under reduced pressure. The resultant residue was purified by preparative TLC (chloroform-methanol-aqueous ammonia (100:10:1)) to afford 28 mg of the title compound.

### Physicochemical properties of this compound

(1) Mass spectrum (ESI): m/z 858 (M+H)⁺
(2) ¹H NMR spectrum (400 MHz, CDCl₃ δ (ppm): 0.88 (d, 3H), 1.18 (d, 3H), 1.21 (d, 3H), 1.53 (m, 2H), 2.05 (s, 3H), 2.24 (s, 3H), 2.33 (dd, 1H), 2.50 (s, 6H), 3.05 (m, 2H), 3.18 (br t, 1H), 3.26 (dq, 1H), 3.36 (br d, 1H), 3.46 (dd, 1H), 3.53 (s, 3H), 3.97 (m, 2H), 4.45 (d, 1H), 5.00 (ddq, 1H), 5.15 (br s, 1H), 5.23 (br t, 1H), 5.75 (dd, 1H), 5.84 (br d, 1H), 7.23 (d, 1H), 7.54 (dd, 1H), 7.68 (dd, 1H), 8.02 (d, 1H), 8.08 (d, 1H), 8.78 (d, 1H), 9.67 (s, 1H).

### Reference Example Antibacterial activity of compound represented by formula (III)

The compound obtained in the present invention was measured for its in vitro antibacterial activity by a broth microdilution method in accordance with a CLSI method (formerly NCCLS method, M31-A2) (Performance Standards for Antimicrobial Disk and Dilution Susceptibility Tests for Bacteria Isolated from Animals; Approved Standard-Second Edition NCCLS M31-A2 Vol. 22 No. 6 2002). A medium composition used in the measurement is shown below.

A test drug solution in which a test drug was dissolved at 1,280 µg/mL in ethanol was diluted 10-fold with the liquid medium shown below. The resultant test drug solution was further subjected to two-step dilution with the liquid medium shown below to prepare a test drug solution at each concentration level. The thus prepared test drug solution at each concentration level was dispensed into a 96-well microplate at 100 µL/well, and a test bacterial strain was inoculated at about 5×10⁴ CFU/well.

After having been cultured in the presence of 5% CO₂ gas at 37°C for 20 to 24 hours, the test bacterial strain was visually observed for the presence or absence of its growth. A minimum drug concentration completely inhibiting the growth of the test bacterial strain was defined as a minimum inhibitory concentration (hereinafter referred to as MIC).

### Liquid medium

| | |
|---|---|
| BBL Mueller Hinton II broth (Nippon Becton Dickinson Company, Ltd.) | 22.0 g |
| Lysed horse blood* | 20 mL |
| NAD (Wako Pure Chemical Industries, Ltd.) | 0.2 g |
| Purified water | 1,000 mL |

| | |
|---|---|
| *Lysed horse blood | |

| | |
|---|---|
| Saponin (Kanto Chemical Co., Inc.) | 2.0 g |
| Purified water | 10 mL |
| Defibrinated horse blood (Japan Lamb) | 100 mL |

Saponin was dissolved with purified water. The solution was sterilized and then added to defibrinated horse blood.

**TABLE 1**

| MICs (µg/ml) of compounds represented by formulae (IIIa) and (IIIb) and existing animal antibacterial agents | | | | | | | | | |
|---|---|---|---|---|---|---|---|---|---|
| Species | Strain | TS | AIV | TMS | TLM | GTM | JM | (IIIa) | (IIIb) |
| *P. multocida* | 11272R | 64 | 128 | 32 | 16 | 4 | 16 | 4 | 4 |
| (bovine) | 11325-1R | 16 | 128 | 16 | 4 | 1 | 8 | 2 | 2 |
| | 11787-24R1 | 32 | 128 | 16 | 8 | 2 | 32 | 4 | 4 |
| | ATCC43019 | 64 | 128 | 32 | 16 | 4 | 64 | 4 | 4 |
| (swine) | 11587-3R | 64 | 256 | 16 | 16 | 4 | 32 | 4 | 4 |
| | 11745-15R | 64 | 256 | 16 | 8 | 2 | 32 | 4 | 4 |
| | ATCC43137 | 64 | 256 | 16 | 16 | 2 | 32 | 2 | 4 |
| *A. pleuropneumoniae* | 11796-1R | 32 | 32 | 16 | 16 | 4 | 16 | 4 | 4 |
| | 11684-7R | 256 | 64 | 128 | 64 | 16 | 64 | 16 | 32 |
| | 10895-2R | 16 | 32 | 16 | 16 | 4 | 32 | 4 | 4 |
| | 11804-2R | 32 | 32 | 16 | 16 | 4 | 8 | 4 | 8 |
| | ATCC27089 | 32 | 32 | 16 | 32 | 4 | 16 | 4 | 4 |
| *M. haemolytica* | 11443R | 16 | 32 | 16 | 16 | 2 | 16 | 2 | 4 |
| | 11467-2R | 128 | 64 | 8 | 32 | 2 | 32 | 4 | 4 |
| *H. somni* | 11321R | 1 | 1 | 1 | 1 | 0.5 | 2 | 0.5 | 0.5 |
| | 11423-3R | 4 | 8 | 4 | 1 | 0.5 | 8 | 1 | 1 |
| *H. parasuis* | 11510-1-2R | 4 | 16 | 1 | 4 | 0.25 | 8 | 0.25 | 0.5 |
| | 11640-1R | 8 | 32 | 16 | 8 | 1 | 32 | 2 | 4 |

### INDUSTRIAL APPLICABILITY

The present invention has allowed a novel production method and intermediate for an animal antibacterial agent having strong antibacterial activities against main pathogens of bacterial respiratory tract infections problematic in livestock animals such as cattle and swine to be provided.

## Claims

1. A compound, which is represented by the following formula (I), or a pharmacologically acceptable salt thereof: 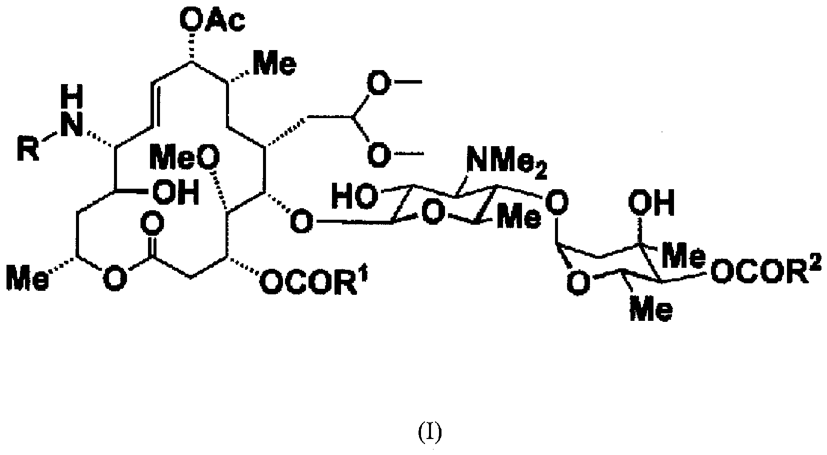 where R represents a lower alkyl group, an alkyl group that may have a substituent, an aralkyl group that may have a substituent, or a heterocyclic alkyl group that may have a substituent, R¹ represents a methyl group or an ethyl group, and R² represents an ethyl group or an isobutyl group.

2. A compound, which is represented by the following formula (II), or a pharmacologically acceptable salt thereof: 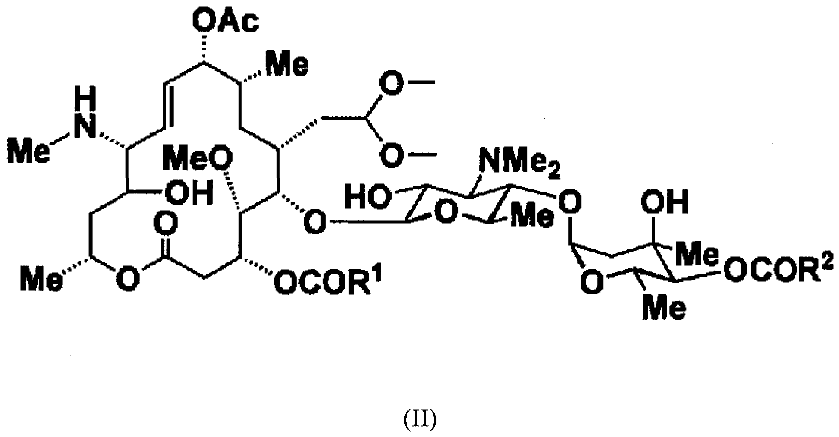 where R¹ represents a methyl group or an ethyl group, and R² represents an ethyl group or an isobutyl group.

3. A compound according to claim 2, wherein R¹ and R² each represent an ethyl group.

4. A compound according to claim 2, wherein R¹ represents a methyl group, and R² represents an isobutyl group.

5. A production method for a compound represented by the following formula (III), comprising subjecting a compound represented by the formula (I) to react with 3-(quinolin-4-yl)propylaldehyde and subjecting the resultant to a reduction reaction: 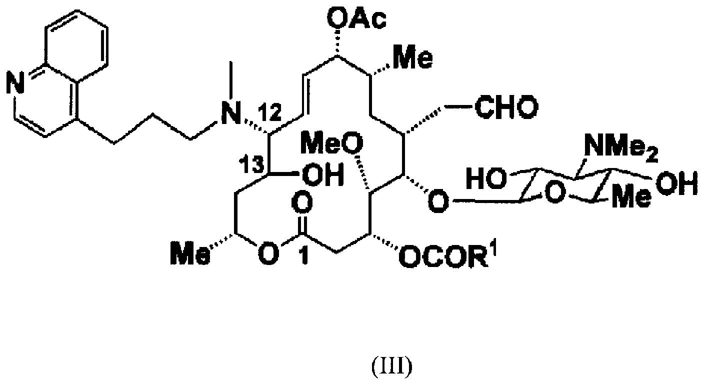 where R¹ represents a methyl group or an ethyl group.

6. A production method for the compound represented by the formula (I) as defined in claim 1, comprising allowing a compound represented by the formula (IV) to react with an organophosphorous reagent and subsequently with an aldehyde reagent and subjecting the resultant to a reduction reaction: 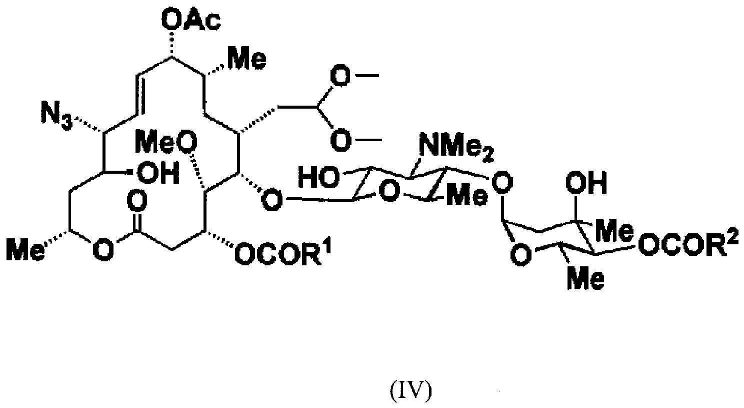 where R¹ represents a methyl group or an ethyl group, and R² represents an ethyl group or an isobutyl group.

7. A production method for the compound represented by the formula (II) as defined in any one of claims 2 to 4, comprising allowing a compound represented by the formula (IV) to react with an organophosphorous reagent and subsequently with paraformaldehyde and subjecting the resultant to a reduction reaction.
